# FASCICULE DE BREVET EUROPEEN

(11) **EP 0 323 796 B1**
(45) Date de publication et mention de la délivrance du brevet: **31.08.1994**
(21) Numéro de dépôt: 88500086.9
(22) Date de dépôt: 21.09.1988
(51) Int. Cl.: A61B 17/54, A45D 29/18

(54) **Dispositif coupe-durillons graduable**
Verstellbares Werkzeug zum Abschneiden von Schwielen
Adjustable callus cutter

(30) Priorité: 28.12.1987 ES 8704041 U
(43) Date de publication de la demande: 12.07.1989
(73) Titulaire: Pascual Gombau, Amado, E-08211 Castellar del Valles Barcelona (ES)
(72) Inventeur: Pascual Gombau, Amado, E-08211 Castellar del Valles Barcelona (ES)
(74) Mandataire: Comas Carreras, Jaime

(56) Documents cités:
- EP-A- 0 202 139
- CH-A- 83 208
- FR-A- 919 249
- GB-A- 658 916
- US-A- 2 534 666

## Description

Le présent brevet d'invention concerne un dispositif coupe-durillons qui offre plusieurs avantages par rapport à toutes les exécutions similaires existant sur le marché. En effet, il s'agit maintenant d'un outil de pédicure ou de manucure permettant l'utilisation de lames conventionnelles et possédant des moyens pour régler à volonté la profondeur de la taille, étant en outre muni d'autres éléments utilisables pour le traitement de pieds, tel que lime, grattoir, un repoussoir et un coupe-peaux, tout cela constituant une unité très pratique aussi bien pour le particulier que pour le professionnel.

La technique actuelle connaît déjà divers dispositifs coupants pour ladite fonction, comme on peut vérifier dans les brevets US-A-2534666 (coupe-durillons) (Grisso), FR-A-919249 (Rasoir mécanique) (de Raymond), GB-A-658916 (Dispositif lime à ongles et racleur de durillons) (Legge) et EP-A-0202139 (Outil de manucure) (Tula Gil), mais aucune de ces réalisations ne coïncident structurellement avec l'objet de la présente invention. En effect, les trois premiers brevets protègent des coupeurs formés par une lame au tranchant parallèle à l'axe longitudinal du dispositif, c'est-à-dire qu'il a une action latérale. Il n'existe pas de possibilité de graduer la profondeur de la coupe. Dans le dernier brevet, il ne se trouve même pas une lame mais seulement un coupe-cuticules d'extrémité, ayant un tranchant en "V", non approprié pour couper directement des durillons ou des callosités.

En ce qui concerne le brevet CH-A-83208 Coupe cors (Werner) également connu, il n'offre pas non plus une constitution analogue à celle de la présente invention. Celui-ci se rapporte à une lame dentée. Cette partie dentée est parallèle à l'axe longitudinal du manche. Il n'existe pas de possibilité de graduation de la profondeur de la coupe, car l'on de fait référence qu'à la variation de la courbe de la lame pour l'adapter à son support. Il n'y a pas de fenêtres transversales pour que sorte le tranchant coupant et ladite lame n'est pas du type conventionnelle. Il n'existe pas non plus d'ailettes de centrage.

Le nouveau coupe-durillons de la présente invention est défini dans la revendication 1. Il est du type constitué par une poupée unie à un manche et pourvue d'une lame coupante, est caractérisé en ce que en sa poupée en tôle concave-convexe qui fait corps avec le manche correspondant, apparaissent, disposées transversalement à l'axe longitudinal de ce dispositif, deux fenêtres transversales parallèles destinées à la sortie des tranchants de la lame rectangulaire correspondante du type conventionnel appliquée sur le fond concave de la poupée, lesquelles sont situées sur des lignes qui sont aussi parallèles à ladite lame, qui se trouve au centre de la propre poupée, figurant également deux ailettes courbées de la propre tôle et destinées à pénétrer aussi bien par les orifices latéraux que présente cette lame que par d'autres orifices opérés avec le même alignement transversal dans une tôle de serrage, également doublée rectangulaire prévue pour serrer ou libérer cette lame pour que ses tranchants puissent avancer ou reculer, pour ainsi varier la profondeur de la taille, soit la grosseur de la peau coupée.

Pour mieux comprendre la présente description, deux feuilles de dessins y sont jointes, dans lesquelles, simplement à titre d'exemple et non limitatif, est représenté un cas pratique d'exécution d'un dispositif coupe-durillons aux caractéristiques générales exposées.

Dans ces dessins:
La Fig. 1 est une vue du côté interne de' la poupée de taille dudit dispositif;
La Fig. 2 correspond à une vue éclatée sectionnée des éléments composant la propre poupée;
La Fig. 3 équivaut à la précédente, mais à présent avec toutes les pièces assemblées, en position de travail, et en section par la ligne III - III de la fig. 1;
La Fig. 4 est une vue complète, partiellement sectionnée, du propre coupe-durillon; et
La Fig. 5 est un détail qui montre l'adaptation d'un des accessoires audit dispositif.

L'objet de cette demande est constitué, pour la forme usuelle, par le manche (1) et la poupée concave-convexe (2), le premier desquels est, dans ce cas, creux pour permettre la fonction qui sera expliquée plus bas.

Au centre de la poupée (2) (en tôle) apparaît le col (3), à intérieur fileté, aux deux côtés duquel ont été formés, par taille et pliage de la propre tôle, des ailettes (4), qui restent sensiblement en angle droit par rapport au fond de cette tôle (2), dans laquelle de plus on été pratiquées les fenêtres parallèles transversales (5), destinées à la sortie des deux tranchants de la lame rectangulaire (6), qui est de type conventionnel et qui est soutenue serrée par l'effet de la pièce de serrage (7), également rectangulaire et concave-convexe, portant les orifices (8) et un col central (9), qui coincide avec le col fileté (3) cité plus haut. Des fenêtres citées (5), celle du bout laisse sortir le tranchant coupant qui doit agir sur la peau entre les doigts des mains ou des pieds, tandis que la fenêtre intérieure laisse passer le tranchant prévu pour couper les peaux ou callosités des surfaces restantes des mains ou des pieds.

Pour fixer les composants (6) et (7) on utilise la vis (10), qui fait partie du bouton (11). Grâce à cette vis, ces pièces ne peuvent jamais s'échapper ou se lâcher, comme c'est le cas des réalisations dans lesquelles la lame est montée à l'extérieur.

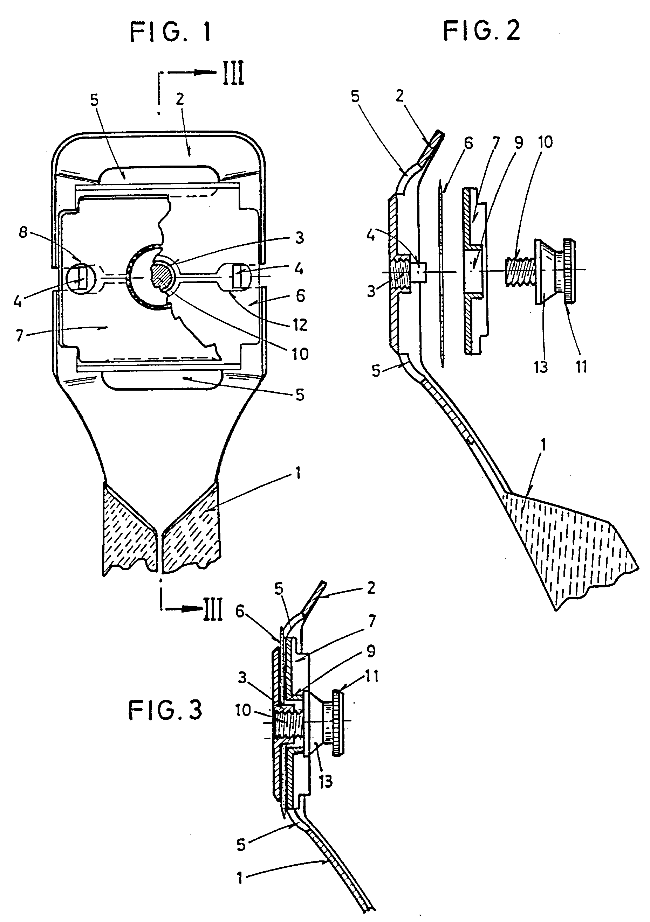

Comme on peut voir clairement dans la Fig. 1, les ailettes (4) pénètrent tout d'abord dans les orifices normaux (12) de la lame standard (6) et, ensuite, dans les orifices (8) de la tôle de serrage (7), avec quoi on arrive à immobiliser complètement les deux pièces, contre le fond concave de la poupée (2), ce qui fait que les tranchants de ladite lame (6), toujours courbée par l'action de la tôle de serrage (7), dépassent exactement par les fenêtres (5).

Cette sortie peut être graduée, c'est-à-dire la profondeur de la taille (grosseur plus ou moins grande de la peau coupée) dépend de l'avancement (vissage) ou recul (dévissage) du bouton (11) (Fig. 3), qui est celui qui, à travers sa butée (13), fait avancer ou laisse reculer la tôle (7) et, donc, la lame (6) pour qu'elle en fasse tout autant à travers ces fenêtres (5).

L'effet élastique ou de ressort pour cette graduation est à la charge de la propre lame (6), qui est courbée ou appliquée par force plus ou moins sur le fond de la poupée (2) par la pression sur la tôle de serrage (7) et qui réagit et recule lorsque ledit bouton (11) est dévissé.

Comme il est évident, l'avantage qu'offre cette nouvelle poupée réside en ce que maintenant il est possible d'employer des lames (6) du marché, c'est-à-dire des lames rectangulaires, car les fenêtres (5) et la pièce de serrage (7) ont été adaptées à cette forme, en ayant obtenu une retenue parfaite et le centrage desdits composants (6) et (7) grâce aux ailettes (4), qui traversent les ouvertures usuelles (12) de ladite lame (6) et les orifices (8) du moyen de serrage (7).

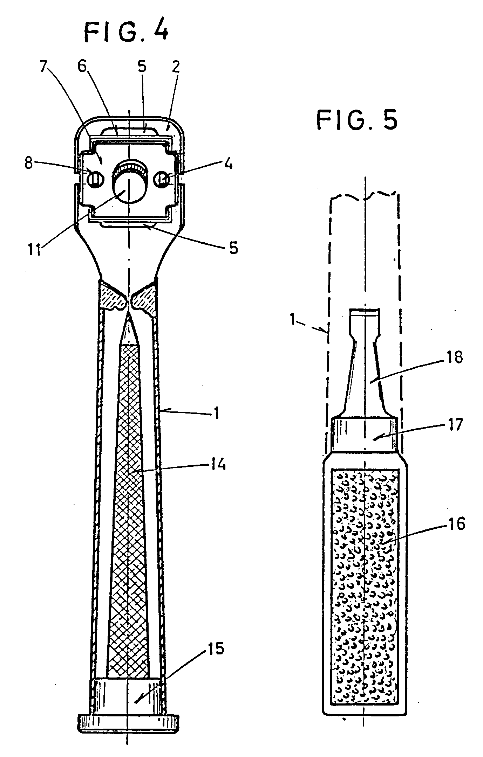

Etant donné que ce coupe-durillons possède un manche (1) creux, il peut être équipé avec des accessoires tels qu'une lime (14), qui est posée dans ce manche et qui est fixée à son embout à l'aide d'un bouchon (15), solidaire de cet élément (14), tout cela comme on peut apprécier à la Fig. 4.

Un autre des accessoires à ajouter est celui que représente la Fig. 5, consistant en un grattoir (16), qui, au moyen du col (17) est fixé à l'embout du manche (1). Ce même grattoir (16) peut recevoir en complément soit un repoussoir (18) soit un coupe-peaux (non visible), étant toujours ces deux adminicules convenablement fixés à ce col (17) (voir Fig. 5).

Les particularités de construction et d'action du coupe-durillons décrit peuvent être résumées aux points suivants:
a) Aussi bien les fenêtres (5) que la ligne qui passe par les ailettes (4) et, donc, par les orifices (12) de la lame (8) et de la tôle de serrage (7) sont transversales à l'axe longitudinal du dispositif, afin de pouvoir appliquer une lame standard rectangulaire, qui reçoit maintenant l'action d'une tôle de serrage également rectangulaire;
b) La profondeur de la coupe est graduable, c'est-à-dire que la grosseur de la peau qui est coupée peut varier, ce qui est possible grâce à ce qu'on emploie une vis qui, en plus de maintenir la tôle de serrage et la lame fixées aux ailettes centreuses citées plus haut superposées sur le fond de la poupée permet que cette tôle de serrage puisse avancer ou reculer selon le vissage et le dévissage de ladite vis, qui porte pour ce le bouton de manoeuvre approprié.
c) En plus de la fonction spécifique du coupe-durillons, ce dispositif peut en remplir auxiliairement quatre autres, savoir: limer, gratter, repousser et couper les peaux, tout cela sans avoir à introduire aucune modification.

Seront indépendants de l'objet de l'invention les matériaux, formes et dimensions des éléments qui forment le coupe-durillons décrit, à condition que les variations introduites n'en affectent pas l'essentialité.

## Revendications

1. Dispositif coupe-durillons graduable, comportant une poupée unie à un manche de manipulation et pourvue d'une lame coupante, dans la poupée en tôle métallique, (2) qui est concave-convexe et forme une unité avec le manche correspondant (1), duquel apparaissent, disposées transversalement à l'axe longitudinal de ce dispositif coupant, deux fenêtres transversales parallèles (5) destinées à la sortie des tranchants d'une lame rectangulaire de type conventionnel (6) appliquée sur la surface concave de la poupée, lesdites fenêtres, étant situées, dans une linge parallèle à ladite lame, ligne qui passe aussi par le centre de la propre poupée, deux ailettes de centrage (4) courbées de la propre tôle (2) pénétrant dans des orifices (12) de la lame (6) aussi bien que par d'autres orifices (8) opérés, dans un alignement transversal pareil, dans une tôle concave-convexe (7) de serrage également rectangulaire, superposée à la lame (6) et prévue pour serrer ou libérer cette lame pour qu'elle puisse avancer ou reculer par propre élasticité au fur et à mesure du vissage et du dévissage d'une vis de serrage (10), qui se déplace à l'intérieur du col fileté (3) situé au centre de la poupée (2) pour ainsi varier la profondeur de la coupe, c'est-à-dire la grosseur de peau coupée par les tranchants de la lame (6) qui dépassent par les fenêtres (5).

2. Dispositif coupe-durillons graduable, selon la revendication précédente, qui est caractérisé en ce que la lame (6) et la tôle concave-convexe (7) sont superposés sur le fond concave de la poupée (2) centrés grâce auxdites ailettes (4) qui sont d'une hauteur supérieure à l'épaisseur de la lame (6) et de la tôle de serrage (7) pour que l'avancement et le recul de cette dernière se fasse dans des conditions optimales de rapidité et de sécurité, ledit réglage ayant toujours comme guide auxiliaire du mouvement un col fileté (3) qui pénètre dans un orifice (9) de ladite pièce de serrage (7) en agissant comme moyen de graduation la propre vis de serrage (10) qui, en plus de s'appuyer sur le filet (3), dispose d'un bouton de manoeuvre (11) avec une butée (13) dûment dimensionnée pour agir sur le col (9) de la tôle mobile, qui, avec son avancement (phase de vissage) et de recul (phase de dévissage), provoque la courbure plus ou moins accusée de la lame.

3. Dispositif coupe-durillons graduable, selon les revendications 1 et 2, qui est caractérisé en ce que son manche creux (1) agit de moyen de contention pour accessoires de pédicure ou de manucure tels qu'une lime (14), un repoussoir (18) et un coupe-peaux éventuel (ciseaux à peaux), la première (14) étant fixée à l'aide d'un bouchon (15) appliqué à l'embout du propre manche (1), tandis que les deuxièmes font partie d'un col (17) qui remplit également la fonction de bouchon et qui, en même temps, fait corps avec un grattoir (16), qui reste à l'extérieur de l'ensemble, en permettant tout cela au dispositif de pouvoir remplir cinq fonctions: couper des durillons, limer, repousser, couper les peaux et gratter.

## Claims

1. An adjustable device for cutting callosities, with a head joined to a handle and fitted with a cutting blade, the head being in sheet metal (2) which is concave-convex and forms a unit with the corresponding handle (1), from which two transversal parallel windows (5) are visible, arranged transversally to the longitudinal axis of this cutting device for extracting the edges of a conventional rectangular blade (6) applied to the concave surface of the head, the windows being located in a line parallel to the blade, this line also passing through the centre of the head itself, two curved centring flanges (4) of the same sheet (2) penetrating the orifices (12) of the blade (6) as well as other orifices (8) in a same transversal alignment operated in a concave-convex clamping sheet (7), which is also rectangular, superposed on the blade (6) and provided as a means of tightening or releasing this blade so that it may be extracted or retracted by its own elasticity as a locking screw (10) is tightened down or unscrewed, this screw being movable inside the threaded neck (3) located in the centre of the head (2) so as to vary the depth of the incision, ie the amount of skin cut by the edges (6) of the blade extracted through the windows (5).

2. An adjustable device for cutting callosities in accordance with the previous claim, which is characterised in that the blade (6) and the concave-convex sheet (7) are superposed on the concave base of the head (2) centred by means of flanges (4) which are at a height greater than the thickness of the blade (6) and the clamping sheet (7) so that the latter may be extracted and retracted under optimum conditions of rapidity and safety, adjustment also being guided by the movement of a threaded neck (3) which penetrates an orifice (9) of the said clamping part (7) by acting as a means of grading the actual locking screw (10), which, as well as bearing on the thread (3), has an operating button (11) with a stop (13) calibrated so as to act on the neck (10) of the movable sheet which, when it is extracted (tightening down phase) and retracted (unscrewing phase), causes greater or lesser curvature of the blade.

3. An adjustable device for cutting callosities in accordance with claims 1 and 2, which is characterised in that its hollow handle (1) serves as a means for containing pedicure or manicure tools such as a file (14), a spatula (18) and possibly a skin cutter (skin scissors), the first (14) being fixed by means of a stop (15) applied to the end of the handle itself (1) whilst the second two items form part of a neck (17) which also serves as a plug and which, at the same time, is integral with a scraper (16), which remains on the outside of the unit, thereby enabling the device to be used for five purposes: cutting callosities, filing, removing skin, cutting skin and scraping.

## Patentansprüche

1. Einstellbares Werkzeug zum Abschneiden von Schwielen, das ein mit einem Griffstück verbundenes und eine Einheit mit diesem bildendes Kopfstück aus Metallblech (2) aufweist, welches konkav-konvex ist und mit einer Schneidklinge versehen ist und welches zwei quer zur Längsachse dieses Schneidwerkzeuges angeordnete querliegende parallele Fenster (5) aufweist, die als Durchlaß für die Schneiden einer die konkave Oberfläche des Kopfstücks aüfgelegten rechtwinkligen Klinge konventionellen Typs (6) dienen, wobei sich diese Fenster auf einer parallel zu der Klinge verlaufenden Linie, die auch durch die Mitte des Kopfstückes selbst geht, befinden, und welches zwei Zentrierstege (4) aufweist, die aus dem Blech (2) selbst gebogen sind und durch Öffnungen (12) der Klinge (6) sowie durch weitere in einer gleicher Ausrichtung quer durch ein ebenfalls rechtwinkliges konkav-konvexes Spannblech (7) ausgebildete Öffnungen (8) hindurchgehen, das über die Klinge (6) gelegt ist und zum Spannen oder Lösen der Klinge dient, damit sich diese in dem Maß vor oder durch Eigenelastizität zurück bewegt, wie eine Spannschraube (10) gespannt oder gelöst wird, welche sich im Inneren eines mit einem Gewinde versehenen, in der Mitte des Kopfstücks (2) befindlichen Halsstücks (3) bewegt, um so die Schnittiefe zu verändern, d.h. die Hautdicke, die von den durch die Fenster (5) hindurchgehenden Schneiden der Klinge (6) abgeschnitten wird.

2. Einstellbares Werkzeug zum Abschneiden von Schwielen nach dem vorstehenden Anspruch, dadurch gekennzeichnet, daß die Klinge (6) und das konkav-konvexe Blech (7) auf dem konkaven Boden des Kopfstücks (2) übereinander gelegt und mittels der Stege (4) zentriert sind, deren Höhe größer als die Dicke der Klinge (6) und des Spannblechs (7) ist, damit die Vor- und Zurückbewegung des letzteren hinsichtlich Schnelligkeit und Sicherheit unter optimalen Bedingungen abläuft und beim Einstellvorgang immer ein Halsstück mit Gewinde (3) zur zusätzlichen Führung der Bewegung durch eine Öffnung (9) des Spannstücks (7) hindurchragt, wobei die Spannschraube (10) als Einstelleinrichtung fungiert, die in das Gewinde (3) eingreift und einen Bedienknopf (11) mit einem Anschlag (13) aufweist, der geeignet zur Einwirkung auf das Halsstück (9) des beweglichen Blechs dimensioniert ist und durch seine Bewegung nach vorn (Einschraubvorgang) und nach hinten (Lösevorgang) eine mehr oder weniger starke Krümmung der Klinge bewirkt.

3. Einstellbares Werkzeug zum Abschneiden von Schwielen nach den Ansprüchen 1 und 2, dadurch gekennzeichnet, daß sein hohler Griff (1) als Behälter für Pediküre- oder Manikürezubehör, wie beispielsweise eine Feile (14), ein Rückschieber (18) und eventuell ein Hautschneider (Hautschere) ausgebildet ist, wobei erstere (14) mittels eines am Endstück des Griffs (1) angebrachten Stopfens (15) befestigt wird, die beiden letzteren hingegen einen Teil mit einem ebenfalls als Stopfen dienenden Halsstück (17) bilden, das gleichzeitig Teil einer außerhalb der Anordnung verbleibenden Raspel ist, all dieses derart daß das Werkzeug fünf Funktionen erfüllen kann: Schwielen abschneiden, feilen, zurückdrücken Haut schneiden und raspeln.
